# EUROPÄISCHE PATENTSCHRIFT

(11) **EP 3 413 775 B1**
(45) Veröffentlichungstag und Bekanntmachung des Hinweises auf die Patenterteilung: **15.07.2020**
(21) Anmeldenummer: 17703423.8
(22) Anmeldetag: 06.02.2017
(51) Int. Cl.: A61B 1/00, A61B 1/018, A61B 1/307, A61B 1/07

(54) **CHIRURGISCHES INSTRUMENT UND VERFAHREN ZUM HERSTELLEN EINES CHIRURGISCHEN INSTRUMENTS**
SURGICAL INSTRUMENT AND METHOD FOR PRODUCING A SURGICAL INSTRUMENT
INSTRUMENT CHIRURGICAL ET PROCÉDÉ DE FABRICATION D'UN INSTRUMENT CHIRURGICAL

(30) Priorität: 09.02.2016 DE 102016201905
(43) Veröffentlichungstag der Anmeldung: 19.12.2018
(73) Patentinhaber: Olympus Winter & Ibe GmbH, 22045 Hamburg (DE)
(72) Erfinder: KIEDROWSKI, Gregor, 22397 Hamburg (DE)
(74) Vertreter: Seemann & Partner Patentanwälte mbB
(86) Internationale Anmeldenummer: PCT/EP2017/052556
(87) Internationale Veröffentlichungsnummer: WO 2017/137357

(56) Entgegenhaltungen:
- EP-A1- 2 283 767
- EP-A2- 1 776 918
- DE-A1- 3 206 381
- US-A1- 2005 182 353

## Beschreibung

Die Erfindung betrifft ein chirurgisches Instrument mit einem langgestreckten Schaftrohr, an welches sich ein Hauptkörper anschließt, und mit einem Anschlusskörper, wobei der Anschlusskörper mit einem proximalen Endbereich des Schaftrohrs gekoppelt ist, und wobei in einem Innenraum des Schaftrohrs zumindest ein Arbeitskanalrohr angeordnet ist, welches sich in Längsrichtung des Schaftrohrs erstreckt. Ferner betrifft die Erfindung ein Verfahren zum Herstellen eines solchen chirurgischen Instruments.

In der Urologie werden Uretheroskope als chirurgische Instrumente für endoskopische Arbeiten im Nierenbecken eingesetzt. Um endoskopisch in diesen Bereich zu gelangen, wird das Uretheroskop von außen durch die Urethra (Harnröhre) in die Blase vorgeschoben und von dort in den Ureter (Harnleiter) eingeführt. Das Schaftrohr eines Uretheroskops hat vielfach eine Länge von 400 mm, sein Durchmesser beträgt in etwa 3 bis 4 mm. Um dem Schaftrohr bei dieser extrem langen und dünnen Konfiguration die notwendige Stabilität zu verleihen, handelt es sich üblicherweise um ein Metallrohr. Es sind Uretheroskope als chirurgische Instrumente mit starrem oder halbstarrem Schaft bekannt.

In einem von dem Schaftrohr umschlossenen Innenraum sind wenigstens eine Beobachtungseinrichtung, eine Vielzahl von Lichtleitern zur Beleuchtung des Operationsfeldes sowie zumindest ein, vielfach zwei, Arbeitskanäle vorhanden. Die Arbeitskanäle erstrecken sich jeweils in einem Arbeitskanalrohr. Durch sie werden Instrumente, beispielsweise Katheter, Zangen, Lithotripter oder dergleichen, in das Operationsgebiet geführt. Ein Uretheroskop ist beispielsweise aus der DE 10 2004 059 255 B3 bekannt.

Aus DE 32 06 381 A1 ist ein Endoskop bekannt, welches einen Schaft mit einem flexiblen Schaftteil aufweist. Der Schaft ist durch einen Drehansatz geführt, der aus zwei gegeneinander verdrehbaren Teilen besteht, von denen der eine Teil am Schaft und der andere Teil am starren proximalen Endstück des flexiblen Endoskops befestigt ist.

Aus US 2005/0182353 A1 ist ein Endoskop bekannt, dessen Schaft aus einer ersten Röhre und einer zweiten Röhre aufgebaut ist. Die zweite Röhre ist kein geschlossenes Rohr und ist auf die Außenseite der ersten Röhre beispielsweise aufgeschweißt. Ein Verbindungsstück umgibt den Schaft. Die Verbindung zwischen diesen beiden Bauteilen wird mittels eines Klebstoffs hergestellt.

Aus EP 1 776 918 A2 ist ein Endoskop bekannt, dessen Endoskopschaft aus einem ersten Rohr und aus einem darin geführten zweiten Rohr aufgebaut ist. Zwischen dem ersten Rohr und dem zweiten Rohr ist ein Kanal vorhanden, durch den Lichtleiter geführt werden. Der Zwischenraum zwischen den beiden Rohren wird mit einem Klebstoff vergossen.

Aus EP 2 283 767 A1 ist ein Endoskop mit einem Schaft bekannt, der aus einem Außenrohr und einem innerhalb dieses Außenrohrs angeordneten Innenrohr besteht. Das Außenrohr und das Innenrohr sind in Axialrichtung des Schafts relativ zueinander bewegbar am Instrumentengehäuse befestigt. Hierzu ist das Innenrohr über eine flexible Membran an dem inneren Gehäuseteil gehalten. Bei der flexiblen Membran handelt es sich beispielsweise um eine korrosionsbeständige Metallmembran. Die Verbindung der Membran mit den angrenzenden Bauteilen erfolgt fluiddicht, beispielsweise durch Verlöten, Verkleben oder Verschweißen. Zusätzlich ist ein Dichtungselement zwischen dem äußeren Gehäuseteil und dem Außenrohr vorgesehen. Die zwischen dem Innenrohr und dem inneren Gehäuseteil angeordnete Membran kann ebenfalls durch Verlöten, Verkleben oder Verschweißen fluiddicht ausgebildet sein.

Nach dem chirurgischen Einsatz werden die Uretheroskope aufbereitet, also gereinigt und desinfiziert. Im Rahmen der Desinfektion werden die chirurgischen Instrumente vielfach in einem Autoklaven behandelt. Um ein Eindringen der Reinigungs- bzw. Aufbereitungsflüssigkeit oder des zum Autoklavieren verwendeten Dampfs ins Innere des chirurgischen Instruments zu verhindern, sind die Arbeitskanäle am proximalen Ende des Uretheroskops mit Hilfe eines Dichtungssatzes gegenüber dem Hauptkörper des Uretheroskops abgedichtet. Die Zuverlässigkeit dieser Dichtungen lässt jedoch im Laufe der Zeit nach. Daher müssen die Dichtungen in regelmäßigen Abständen überprüft und falls erforderlich ausgetauscht werden.

Herkömmliche Uretheroskope sind aus diesem Grund so konstruiert, dass die Zugänglichkeit der Dichtungselemente gewährleistet ist.

Dies bedingt jedoch einen nicht unerheblichen konstruktiven Aufwand. Außerdem ist die regelmäßig durchzuführende Wartung der Dichtungselemente zeit- und kostenintensiv.

Es ist eine Aufgabe der Erfindung, ein chirurgisches Instrument sowie ein Verfahren zum Herstellen eines chirurgischen Instruments anzugeben, wobei das chirurgische Instrument einfach aufzubereiten, wartungsfreundlich und konstruktiv einfach sein soll.

Die Aufgabe wird entsprechend Anspruch 1 gelöst durch ein chirurgisches Instrument mit einem langgestreckten Schaftrohr, an welches sich ein Hauptkörper anschließt, und mit einem Anschlusskörper, wobei der Anschlusskörper mit einem proximalen Endbereich des Hauptkörpers gekoppelt ist, und wobei in einem Innenraum des Schaftrohrs zumindest ein Arbeitskanalrohr angeordnet ist, welches sich in Längsrichtung des Schaftrohrs erstreckt, wobei ein proximaler Endbereich des Arbeitskanalrohrs innerhalb des Anschlusskörpers aufgenommen und gegenüber diesem abgedichtet ist, wobei das chirurgische Instrument dadurch fortgebildet ist, dass das Arbeitskanalrohr mittels eines schaftförmigen Dichtungsansatzes mit dem Hauptkörper verbunden ist und der schaftförmige Dichtungsansatz einen Mantelabschnitt und einen Verbindungsabschnitt umfasst, wobei der Verbindungsabschnitt in einer radialen Richtung eine größere Materialstärke aufweist als der Mantelabschnitt und der Mantelabschnitt mittels zumindest einer sich zumindest näherungsweise in Längsrichtung erstreckenden linienförmigen Durchschweißverbindung mit dem Arbeitskanalrohr verbunden ist.

Vorteilhaft ist der Arbeitskanal direkt gegenüber dem Anschlusskörper und nicht wie bei herkömmlichen Instrumenten gegenüber dem Hauptkörper abgedichtet. Durch diese Maßnahmen wird vermieden, dass während des Aufbereitungsvorgangs Aufbereitungsmedium in innenliegende Frei- oder Hohlräume des chirurgischen Instruments gelangt. Es ist daher einfach und zuverlässig aufzubereiten. Ferner kann vorteilhaft auf die Abdichtung zwischen Arbeitskanalrohr und Schaftrohr sowie zwischen Arbeitskanalrohr und Hauptkörper, insbesondere die dort üblicherweise verwendeten Dichtungsringe, verzichtet werden. Das Arbeitskanalrohr ist bereits gegenüber dem Anschlusskörper abgedichtet. Eine Wartung der Dichtungen entfällt ebenfalls vorteilhaft.

Gemäß einer vorteilhaften Ausführungsform ist das chirurgische Instrument dadurch fortgebildet, dass der proximale Endbereich des Arbeitskanalrohrs gegenüber dem Anschlusskörper mittels einer Klebeverbindung abgedichtet ist.

Durch die vorgesehene Klebeverbindung werden die bei der traditionellen Konstruktion chirurgischer Instrumente in diesem Bereich auftretenden Probleme beseitigt. Bei herkömmlichen Instrumenten werden zum Abdichten des proximalen Endbereichs eines Arbeitskanalrohrs O-Ring-Dichtungen eingesetzt, welche jedoch eine regelmäßige Wartung erfordern. Ferner müssen herkömmliche chirurgische Instrumente so konstruiert sein, dass die Dichtungen zugänglich sind. Indem der proximale Endbereich des Arbeitskanalrohrs bis in den Anschlusskörper fortgesetzt und außerdem innerhalb dessen mittels einer Klebeverbindung verbunden wird, entfallen die geschilderten technischen Notwendigkeiten. Im Ergebnis wird ein wartungsarmes, hervorragend aufzubereitendes und konstruktiv einfaches chirurgisches Instrument bereitgestellt.

Gemäß einer weiteren Ausführungsform ist vorgesehen, dass der Anschlusskörper zumindest einen Zuführkanal umfasst, der mit einem von dem Arbeitskanalrohr umschlossenen Arbeitskanal kommuniziert, insbesondere fluchtet, wobei der Endbereich des Arbeitskanalrohrs in dem Zuführkanal des Anschlusskörpers aufgenommen und mittels einer Klebeverbindung zwischen einer Innenwandung des Zuführkanals und einer Mantelfläche des Arbeitskanalrohrs gegenüber dem Anschlusskörper abgedichtet ist.

Durch die oben genannte bevorzugte Konstruktion, vor allem die direkte Verbindung des Arbeitskanalrohrs mit den Zuführkanälen im Anschlusskörper, wird vermieden, dass im Innenraum des chirurgischen Instruments vorhandene Frei- oder Hohlräume für das Reinigungsmedium zugänglich sind. Derartige Räume sind stets schwierig zu reinigen. Das chirurgische Instrument gemäß Aspekten der Erfindung ist hingegen besonders einfach aufzubereiten.

Das Arbeitskanalrohr ist mittels eines schaftförmigen Dichtungsansatzes mit dem Hauptkörper verbunden.

Der schaftförmige Dichtungsansatz erlaubt vorteilhaft eine mechanisch zuverlässige Ankopplung des Arbeitskanalrohrs. Besonders wichtig und vorteilhaft ist eine solche mechanisch belastbare und zuverlässige Verbindung, wenn es sich um ein chirurgisches Instrument mit halbstarrem, also in gewissem Umfang flexiblem, Schaft handelt. Dies gilt insbesondere, wenn das chirurgische Instrument ein Uretheroskop ist.

Bevorzugt ist vorgesehen, dass der schaftförmige Dichtungsansatz an seiner Innenwandung mit einem proximalen Endbereich des Arbeitskanalrohrs und an seiner äußeren Mantelfläche mit einem proximalen Endbereich des Hauptkörpers verbunden ist.

Bei dem Arbeitskanalrohr handelt es sich um ein Bauteil mit geringer Wand- bzw. Materialstärke. Bei chirurgischen Instrumenten mit halbstarrem Schaft, insbesondere bei Uretheroskopen, ist das Arbeitskanalrohr bei einer Biegebewegung des Schafts nicht zu vernachlässigenden Druck- und Zugbelastungen ausgesetzt. Um die erforderliche mechanische Stabilität der Verbindung zwischen dem Arbeitskanalrohr und dem Hauptkörper bereitzustellen, ist vorgesehen, dass der schaftförmige Dichtungsansatz einen Mantelabschnitt und einen Verbindungsabschnitt umfasst, wobei der Verbindungsabschnitt in einer radialen Richtung eine größere Materialstärke aufweist als der Mantelabschnitt und der Mantelabschnitt mittels zumindest einer sich zumindest näherungsweise in Längsrichtung erstreckenden linienförmigen Durchschweißverbindung mit dem Arbeitskanalrohr verbunden ist.

Bevorzugt handelt es sich bei der linienförmigen Durchschweißverbindung um eine lasergeschweißte Verbindung.

In einem Laserschweißverfahren können Bauteile geringer Materialstärke zuverlässig miteinander verbunden werden. Der schaftförmige Dichtungsansatz stellt für die Verbindung zwischen dem Dichtungsansatz und dem Arbeitskanalrohr einen Mantelabschnitt bereit. Dieser Mantelabschnitt hat beispielsweise die Form eines Hohlzylinders mit geringer Wandstärke. Der Verbindungsabschnitt hat ebenfalls beispielsweise die Form eines Hohlzylinders, jedoch im Vergleich zum Mantelabschnitt mit erheblich größerer Wandstärke und geringerer Länge. So ist gewährleistet, dass im Verbindungsabschnitt des schaftförmigen Dichtungsansatzes genügend Material für eine zuverlässige, insbesondere mit einem (Laser-)Schweißverfahren realisierte, Verbindung zu dem Hauptkörper vorhanden ist. Der Mantelabschnitt ist hingegen geringfügig flexibel und folgt so in gewissem Umfang der Verformung des Arbeitskanalrohrs. Der Mantelabschnitt und der Verbindungsabschnitt des Dichtungsansatzes sind stoffeinteilig. Es handelt sich also mit anderen Worten bei dem Dichtungsansatz beispielsweise um ein aus einem Rohling gedrehtes Werkstück.

Gemäß einer weiteren Ausführungsform ist das chirurgische Instrument dadurch fortgebildet, dass proximal des Dichtungsansatzes zwischen Hauptkörper und Anschlusskörper ein an den Dichtungsansatz angrenzender Hohlraum im Anschlusskörper vorhanden ist, wobei der proximale Endbereich des Arbeitskanalrohrs mit einer Klebeverbindung mit dem Anschlusskörper verbunden ist und der Hohlraum mit einem Dichtmittel, insbesondere mit einem Dichtungsring, das zwischen einer äußeren Mantelfläche des Arbeitskanalrohrs und dem Anschlusskörper vorhanden ist, von der Klebeverbindung getrennt ist, wobei die Klebeverbindung durch einen unter Einwirkung von Wärme aushärtenden Klebstoff bereitgestellt wird.

Wenn der Klebstoff durch Einwirkung von Wärme ausgehärtet wird, erwärmt sich ebenfalls die im Hohlraum vorhandene Luft. Bei diesem Hohlraum handelt es sich um einen aus dem Innenraum des Arbeitskanals heraus nicht zugänglichen Hohlraum. Der Dichtungsring verhindert, dass sich während dieses Vorgangs eine Druckbelastung (durch die sich ausdehnende Luft) auf die Klebeverbindung ergibt. Diese könnte Mikrokanäle in der noch nicht vollständig ausgehärteten Klebstoffmasse erzeugen, was die hermetische Dichtheit der Klebeverbindungen infrage stellen könnte.

Gemäß einer weiteren vorteilhaften Ausführungsform ist das chirurgische Instrument dadurch fortgebildet, dass zumindest eine der folgenden Verbindungen eine lasergeschweißte Verbindung ist:
- die Verbindung zwischen dem Hauptkörper und dem Anschlusskörper,
- die Verbindung zwischen dem Dichtungsansatz und dem proximalen Endbereich des Hauptkörpers,
- die Verbindung zwischen dem Schaftrohr und dem Hauptkörper.

Insbesondere ist vorgesehen, dass die mehreren linienförmigen lasergeschweißten Verbindungen, welche den schaftförmigen Dichtungsansatz an seiner Innenwandung mit einer äußeren Mantelfläche des proximalen Endbereichs des Arbeitskanalrohrs verbinden, sich in Längsrichtung des Arbeitskanalrohrs erstrecken und gleichverteilt entlang seines Umfangs angeordnet sind. So wird eine zuverlässige und mechanische Verbindung zwischen dem Arbeitskanalrohr und dem schaftförmigen Dichtungsansatz geschaffen.

Der schaftförmige Dichtungsansatz kann Druck- und/oder Zugkräfte, die auf das Arbeitskanalrohr wirken, aufnehmen und diese in den Hauptkörper bzw. das Schaftrohr einleiten. Dies ist insbesondere für chirurgische Instrumente mit einem halbstarren langgestreckten Schaftrohr von Bedeutung. Das Arbeitskanalrohr ist regelmäßig nicht entlang einer für die Verbiegung des Schaftrohrs geltenden neutralen Linie geführt, so dass es Druck- und/oder Zugkräfte aufnehmen muss, wenn das Schaftrohr gebogen wird.

Gemäß einer weiteren Ausführungsform ist der Schaft bzw. das Schaftrohr des chirurgischen Instruments starr oder halbstarr. Es ist also mit anderen Worten in einem vorgegebenen Maße flexibel. Ferner ist das chirurgische Instrument gemäß einer Ausführungsform ein Uretheroskop.

Ferner ist insbesondere vorgesehen, dass die Verbindung zwischen dem Arbeitskanalrohr und dem schaftförmigen Dichtungsansatz stoffschlüssig ist. Eine solche stoffschlüssige Verbindung ist beispielsweise eine Schweißverbindung, insbesondere eine lasergeschweißte Verbindung.

Die Aufgabe wird entsprechend Anspruch 7 ferner gelöst durch ein Verfahren zum Herstellen eines chirurgischen Instruments mit einem langgestreckten Schaftrohr, an welches sich ein Hauptkörper anschließt, und mit einem Anschlusskörper, wobei der Anschlusskörper mit einem proximalen Endbereich des Hauptkörpers gekoppelt ist, und wobei in einem Innenraum des Schaftrohrs zumindest ein Arbeitskanalrohr angeordnet ist, welches sich in Längsrichtung des Schaftrohrs erstreckt, wobei ein proximaler Endbereich des Arbeitskanalrohrs innerhalb des Anschlusskörpers aufgenommen und gegenüber diesem abgedichtet wird, wobei das Verfahren dadurch fortgebildet ist, dass das Arbeitskanalrohr mit einem schaftförmigen Dichtungsansatz und der schaftförmige Dichtungsansatz mit dem Anschlusskörper verbunden wird und der schaftförmige Dichtungsansatz einen Mantelabschnitt und einen Verbindungsabschnitt umfasst, wobei der Verbindungsabschnitt in einer radialen Richtung eine größere Materialstärke aufweist als der Mantelabschnitt und der Mantelabschnitt mit zumindest einer sich zumindest näherungsweise in Längsrichtung erstreckenden linienförmigen Durchschweißverbindung mit dem Arbeitskanalrohr verbunden wird.

Auf das Verfahren zum Herstellen des chirurgischen Instruments treffen gleiche oder ähnliche Vorteile zu, wie sie bereits im Hinblick auf das chirurgische Instrument selbst erwähnt wurden, weshalb auf Wiederholungen verzichtet werden soll.

Gemäß einer vorteilhaften Ausführungsform ist das Verfahren dadurch fortgebildet, dass der proximale Endbereich des Arbeitskanalrohrs gegenüber dem Hauptkörper mittels einer Klebeverbindung abgedichtet wird.

Bei dem Verfahren ist vorgesehen, dass das Arbeitskanalrohr mit einem schaftförmigen Dichtungsansatz und der schaftförmige Dichtungsansatz mit dem Anschlusskörper verbunden wird.

Ferner ist vorgesehen, dass der schaftförmige Dichtungsansatz einen Mantelabschnitt und einen Verbindungsabschnitt umfasst, wobei der Verbindungsabschnitt in einer radialen Richtung eine größere Materialstärke aufweist als der Mantelabschnitt und der Mantelabschnitt mit zumindest einer sich zumindest näherungsweise in Längsrichtung erstreckenden linienförmigen Durchschweißverbindung mit dem Arbeitskanalrohr verbunden wird.

Ein proximaler Endbereich des Schaftrohrs schließt regelmäßig das proximale Ende des Schaftrohrs ein. Der proximale Endbereich erstreckt sich ausgehend von diesem proximalen Ende des Schaftrohrs in Richtung distal. Beispielsweise ist ein solcher proximaler Endbereich wenige Zentimeter lang und nimmt im Verhältnis zur Gesamtlänge des Schaftrohrs wenige Prozent seiner Länge ein, beispielsweise weniger als 10%. Gleiches gilt für den Endbereich des Arbeitskanalrohrs.

Gemäß einer weiteren Ausführungsform ist das Verfahren dadurch fortgebildet, dass die linienförmige Durchschweißverbindung in einem Laserschweißverfahren hergestellt wird.

In einem Laserschweißverfahren können vorteilhaft Bauteile mit geringer Wand- oder Materialstärke zuverlässig miteinander verbunden werden. Aus diesem Grund ist ein solches Verfahren zur Herstellung der Schweißverbindung zwischen dem Arbeitskanalrohr und dem Mantelabschnitt des schaftförmigen Dichtungsansatzes besonders geeignet.

Insbesondere ist ferner vorgesehen, dass zwischen dem proximalen Endbereich, in dem das Arbeitskanalrohr mit der Klebeverbindung mit dem Anschlusskörper verbunden wird, und dem Hohlraum ein Dichtmittel, insbesondere ein Dichtungsring, zwischen einer äußeren Mantelfläche des Arbeitskanalrohrs und dem Anschlusskörper vorgesehen wird, so dass die Klebeverbindung und der Hohlraum voneinander getrennt sind.

Bei dem Dichtungsring handelt es sich beispielsweise um einen O-Ring, welcher zwischen der äußeren Mantelfläche des Arbeitskanalrohrs und dem Hauptkörper angeordnet ist.

Gemäß einer weiteren vorteilhaften Ausführungsform ist vorgesehen, dass für die Klebeverbindung ein durch Einwirkung von Wärme aushärtbarer Kunststoff verwendet wird. Ferner ist insbesondere vorgesehen, dass zum Herstellen der Klebeverbindung der Klebstoff durch Wärmeeinwirkung ausgehärtet wird. Hierbei wird beispielsweise das gesamte chirurgische Instrument auf die zum Aushärten des Klebstoffs notwendige Temperatur erhitzt.

Ferner ist das Verfahren bevorzugt so ausgebildet, dass zumindest eine der folgenden Verbindungen als eine lasergeschweißte Verbindung hergestellt wird:
- die Verbindung zwischen dem proximalen Hauptkörper und dem Anschlusskörper,
- die Verbindung zwischen dem Dichtungsansatz und dem proximalen Endbereich des Hauptkörpers,
- die Verbindung zwischen dem Schaftrohr und dem Hauptkörper.

Insbesondere handelt es sich bei dem Verfahren zum Herstellen eines chirurgischen Instruments um ein Verfahren zum Herstellen eines Uretheroskops.

Weitere Merkmale der Offenbarung werden aus der Beschreibung Ausführungsformen zusammen mit den Ansprüchen und den beigefügten Zeichnungen ersichtlich. Ausführungsformen können einzelne Merkmale oder eine Kombination mehrerer Merkmale erfüllen.

Die Offenbarung wird nachstehend anhand von Ausführungsbeispielen unter Bezugnahme auf die Zeichnungen beschrieben, wobei bezüglich aller im Text nicht näher erläuterten Einzelheiten ausdrücklich auf die Zeichnungen verwiesen wird. Es zeigen:
- Fig. 1: eine schematische Seitenansicht eines Uretheroskops als beispielhaftes chirurgisches Instrument,
- Fig. 2: eine schematisch vereinfachte Längsschnittansicht im Bereich eines proximalen Endbereichs eines Arbeitskanalrohrs eines Uretheroskops gemäß dem Stand der Technik,
- Fig. 3: eine schematisch vereinfachte Längsschnittansicht durch einen proximalen Endbereich eines Arbeitskanalrohrs, welches mit einem schaftförmigen Dichtungsansatz versehen ist,
- Fig. 4: eine schematisch vereinfachte Längsschnittansicht eines solchen Arbeitskanalrohrs mit angesetztem Dichtungsansatz, welches in einem Hauptkörper eingesetzt ist und
- Fig. 5: eine Detaildarstellung eines fertig montierten Uretheroskops als beispielhaftes chirurgisches Instrument in einer schematisch vereinfachten Längsschnittansicht im Bereich des proximalen Endbereichs des Arbeitskanalrohrs.

In den Zeichnungen sind jeweils gleiche oder gleichartige Elemente und/oder Teile mit denselben Bezugsziffern versehen, so dass von einer erneuten Vorstellung jeweils abgesehen wird.

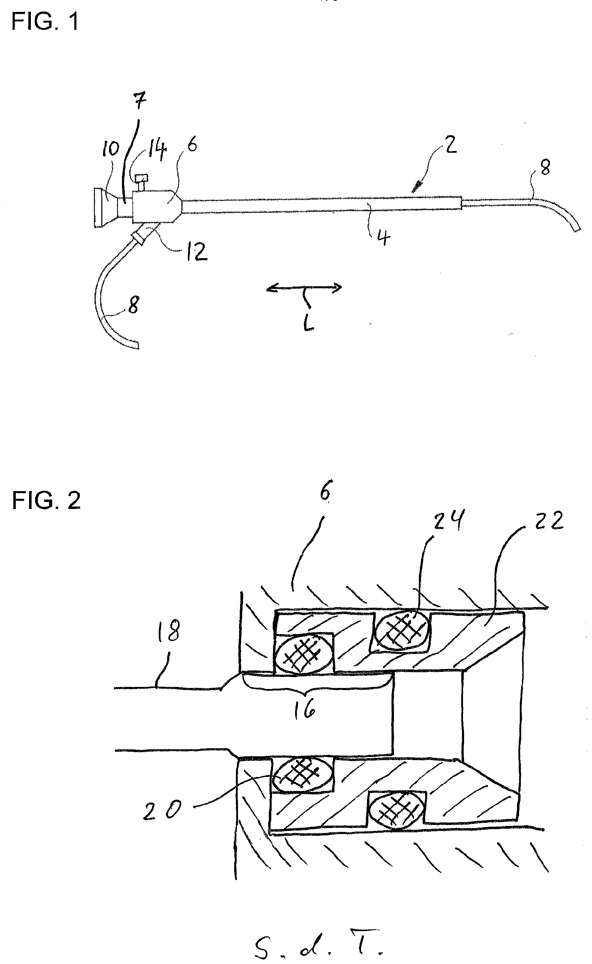

Fig. 1 zeigt in einer schematisch vereinfachten Seitenansicht ein chirurgisches Instrument 2. Beispielhaft ist ein Uretheroskop dargestellt. Das chirurgische Instrument 2 umfasst ein langgestrecktes Schaftrohr 4, welches sich in einer Längsrichtung L erstreckt. Ein proximaler Endbereich des Schaftrohrs 4 ist mit einem Hauptkörper 6 gekoppelt. In proximaler Richtung schließt sich an den Hauptkörper 6 ein Anschlusskörper 7 an. In einem von dem Schaftrohr 4 umschlossenen Innenraum ist ein Arbeitskanalrohr 18 angeordnet (in Fig. 1 nicht sichtbar), welches sich in Längsrichtung L erstreckt. Durch den Innenraum des Arbeitskanalrohrs 18, also den Arbeitskanal, wird ein Behandlungsinstrument 8, beispielsweise ein Katheter, eine Zange oder ein Lithotripter, in ein Operationsgebiet geführt. Ferner erstrecken sich in dem Schaftrohr 4 Lichtleitfasern zur Beleuchtung des Operationsgebiets sowie eine entsprechende Optik, um durch ein Okular 10 das Operationsgebiet zu betrachten. Das Behandlungsinstrument 8 wird durch ein sich an den Anschlusskörper 7 anschließendes Einführungsteil 12 dem chirurgischen Instrument 2 zugeführt. Die zur Beleuchtung des Operationsgebiets vorgesehenen Lichtleitfasern werden über den Lichtleiteranschluss 14 am Hauptkörper 6 versorgt.

Fig. 2 zeigt in einer schematisch vereinfachten Längsschnittansicht den Bereich eines proximalen Endbereichs 16 eines Arbeitskanalrohrs 18 eines Uretheroskops gemäß dem Stand der Technik. Das Arbeitskanalrohr 18 ist in seinem proximalen Endbereich 16 an seiner äußeren Mantelfläche mit Hilfe einer ersten O-Ringdichtung 20 gegenüber einem Zwischenkörper 22 abgedichtet. Dieser Zwischenkörper 22 ist mit einer zweiten O-Ringdichtung 24 gegenüber einer inneren Mantelfläche des Hauptkörpers 6 (welcher lediglich abschnittsweise dargestellt ist) abgedichtet. Die erste und die zweite O-Ringdichtung 22, 24 müssen in regelmäßigen Abständen ausgetauscht bzw. überprüft werden, um eine zuverlässige Abdichtung des Arbeitskanalrohrs 18 gegenüber dem Hauptkörper 6 sicherzustellen.

Demgegenüber ist das chirurgische Instrument 2 gemäß Aspekten der Erfindung so konstruiert, dass das Arbeitskanalrohr 18 direkt in dem Anschlusskörper 7 aufgenommen ist und gegenüber diesem abgedichtet ist. Das chirurgische Instrument 2 gemäß Aspekten der Erfindung wird im Folgenden näher beschrieben.

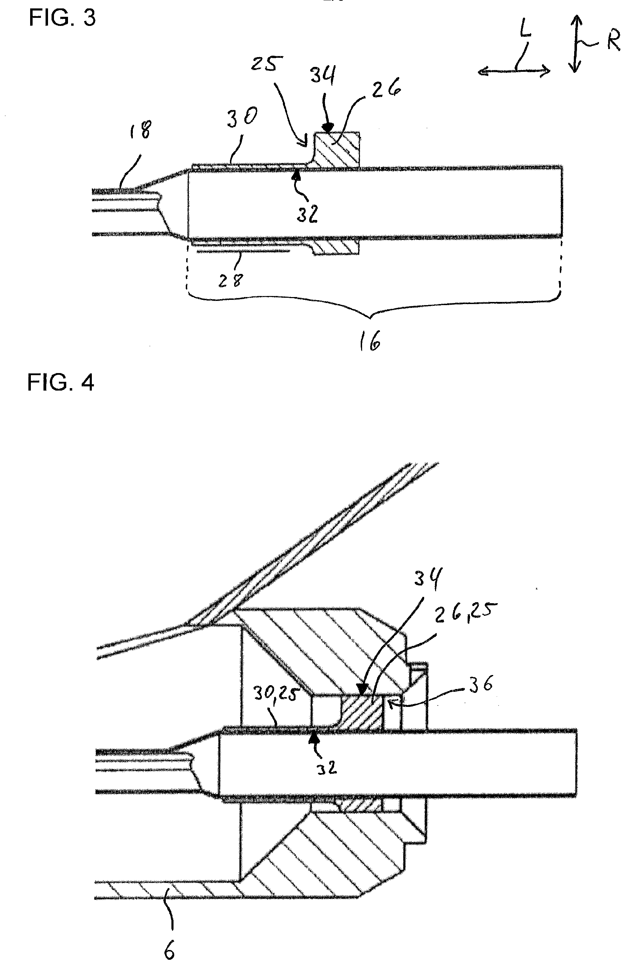

In der schematisch vereinfachten Längsschnittansicht von Fig. 3 ist gezeigt, dass ein proximaler Endbereich 16 des Arbeitskanalrohrs 18 mit einem schaftförmigen Dichtungsansatz 25 verbunden ist. Der schaftförmige Dichtungsansatz 25 umfasst einen Mantelabschnitt 30 und einen Verbindungsabschnitt 26. Der Mantelabschnitt 30 ist mit einer geringen Materialstärke ausgeführt und weist daher eine gewisse Flexibilität in einer Radialrichtung R auf. Die Materialstärke in dieser Radialrichtung R ist bei dem Mantelabschnitt 30 wesentlich geringer als bei dem Verbindungsabschnitt 26. In Längsrichtung L ist der Mantelabschnitt 30 länger als der Verbindungsabschnitt 26. Mantelabschnitt 30 und Verbindungsabschnitt 26 sind Teile des bevorzugt stoffeinteilig/einstückig hergestellten schaftförmigen Dichtungsansatzes 25.

Die Verbindung zwischen dem Dichtungsansatz 25 und dem proximalen Endbereich 16 des Arbeitskanalrohrs 18 erfolgt beispielsweise mit Hilfe einer lasergeschweißten Verbindung, bevorzugt mit Hilfe mehrerer lasergeschweißter Verbindungen. Es werden beispielsweise in Längsrichtung L orientierte Laserschweißlinien 28 gesetzt, von denen in Fig. 3 beispielhaft und schematisch eine angedeutet ist. Die Laserschweißlinien 28 sind entlang des Umfangs des Mantelabschnitts 30 des Dichtungsansatzes 25 bevorzugt gleichverteilt angeordnet, weisen also in Umfangsrichtung gleiche Abstände zueinander auf. Die Laserschweißverbindungen sind bevorzugt Durchschweißverbindungen. Der schaftförmige Mantelabschnitt 30 des Dichtungsansatzes 25, der mit geringer Materialstärke gefertigt ist, wird an die äußere Mantelfläche des Arbeitskanalrohrs 18 in seinem proximalen Endbereich 16 geheftet.

In einer Laserschweißverbindung kann die eingetragene Energie so gut dosiert werden, dass Bauteile mit geringer Materialstärke zuverlässig miteinander verbunden werden können, ohne dass eine Perforation der Bauteile erfolgt. Mit herkömmlichen Verfahren zum Herstellen von Schweißverbindungen ist dies nicht oder nur eingeschränkt möglich.

Das Arbeitskanalrohr 18 mit dem daran befestigten Dichtungsansatz 25 wird anschließend in den Hauptkörper 6 des chirurgischen Instruments 2 verbracht.

In Fig. 4 ist eine schematisch vereinfachte Längsschnittansicht des Arbeitskanalrohrs 18 mit angesetztem Dichtungsansatz 25 gezeigt, wobei das Arbeitskanalrohr 18 in den Hauptkörper 6 eingesetzt ist.

Während der Mantelabschnitt 30 des Dichtungsansatzes 25 an seiner Innenwandung 32 mit dem proximalen Endbereich 16, also der äußeren Mantelfläche des Arbeitskanalrohrs 18, verbunden ist, ist der Verbindungsabschnitt 26 des Dichtungsansatzes 25 an seiner äußeren Mantelfläche 34 mit einem proximalen Endbereich 16 des Hauptkörpers 6 verbunden. So wird eine mechanische Verbindung zwischen dem Hauptkörper 6 und dem Arbeitskanalrohr 18 hergestellt.

Die Verbindung zwischen dem Hauptkörper 6 und dem Arbeitskanalrohr 18 über den Dichtungsansatz 25 erfolgt beispielsweise, indem in der Kehle 36 zwischen dem Dichtungsansatz 25 und einer inneren Mantelfläche des proximalen Endes des Schaftrohrs 4 eine mit Hilfe eines Lasers hergestellte Schweißnaht gesetzt wird. Die in der Kehle 36 gesetzte Schweißnaht kann vollständig oder unterbrochen ausgeführt sein.

Anschließend wird der Anschlusskörper 7 auf den Hauptkörper 6 aufgesetzt, wobei der proximale Endbereich 16 des Arbeitskanalrohrs 18 in dem Anschlusskörper 7 aufgenommen und gegenüber diesem mit Hilfe einer Klebeverbindung 38 abgedichtet wird.

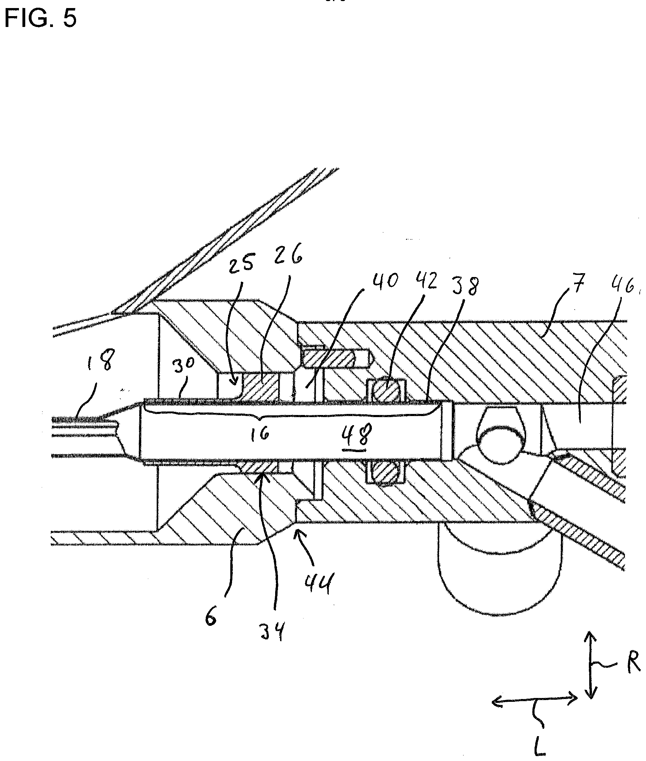

Fig. 5 zeigt eine Detaildarstellung eines fertig montierten chirurgischen Instruments 2 in einer schematisch vereinfachten Längsschnittansicht. Die Klebeverbindung 38 wird umlaufend, sich also entlang des vollständigen Umfangs des Arbeitskanalrohrs 18 erstreckend, ausgeführt. Die Klebeverbindung liegt im proximalen Endbereich 16. Durch die Klebeverbindung wird eine fluidisch dichte Verbindung zwischen dem Anschlusskörper 7 und dem Arbeitskanalrohr 18 hergestellt.

Es wird bevorzugt ein Klebstoff verwendet, der durch Wärmeeinwirkung aushärtet. Um den Klebstoff zum Herstellen der Klebeverbindung 38 auszuhärten, wird beispielsweise das komplette chirurgische Instrument 2 erhitzt.

Proximal des Dichtungsansatzes 25 ist zwischen dem Hauptkörper 6 und dem Anschlusskörper 7 ein an den Dichtungsansatz 25 angrenzender Hohlraum 40 im Anschlusskörper 7 vorhanden. Erhitzt sich die in diesem Hohlraum 40 vorhandene Luft während des Aushärtens der Klebeverbindung 38, so darf der in dem Hohlraum 40 entstehende Überdruck die ggf. noch nicht ausgehärtete Klebeverbindung 38 nicht beeinträchtigen

Bevor die Klebeverbindung 38 vollständig ausgehärtet ist, soll kein Überdruck auf sie wirken. Daher ist zwischen dem Hohlraum 40 und der Klebeverbindung 38 ein Dichtmittel 42 vorgesehen. Beispielsweise handelt es sich bei dem Dichtmittel 42 um einen Dichtungsring (O-Ring). Dieser ist zwischen einer äußeren Mantelfläche des Arbeitskanalrohrs 18 in dessen proximalem Endbereich 16 und dem Anschlusskörper 7 vorhanden. So ist sichergestellt, dass die Klebeverbindung 38 und der Hohlraum 40 voneinander fluidisch getrennt sind.

Der Anschlusskörper 7 wird mit dem Hauptkörper 6 verbunden, indem an der Stoßstelle 44 zwischen diesen beiden Bauteilen umlaufend eine bevorzugt lasergeschweißte Verbindung hergestellt wird. Diese Verbindung ist bevorzugt hermetisch dicht.

Ferner ist insbesondere vorgesehen, dass der Anschlusskörper 7 zumindest einen Zuführkanal 46 umfasst, der mit dem von dem Arbeitskanalrohr 18 umschlossenen Arbeitskanal 48 kommuniziert, insbesondere fluchtet. Durch den Zuführkanal 46 wird in den Arbeitskanal 48 ein Behandlungsinstrument 8 (vgl. Fig. 1) eingeführt und durch das chirurgische Instrument 2 hindurch bis in einen Behandlungsraum geschoben. Der proximale Endbereich 16 des Arbeitskanalrohrs 18 ist in dem Zuführkanal 46 des Anschlusskörpers 7 aufgenommen und mittels der Klebeverbindung 38 zwischen einer Innenwandung des Zuführkanals 46 und einer äußeren Mantelfläche des Arbeitskanalrohrs 18 gegenüber dem Anschlusskörper 7 abgedichtet.

Bei einem Verfahren zum Herstellen eines chirurgischen Instruments 2, insbesondere eines Uretheroskops, werden die in den Fig. 3 bis 5 dargestellten Schritte nacheinander ausgeführt. Zunächst wird der Dichtungsansatz 25 im proximalen Endbereich 16 des Arbeitskanalrohrs 18 angeordnet und bevorzugt mittels Laserschweißverbindungen angeheftet. Anschließend wird das Arbeitskanalrohr 18 in dem Hauptkörper 6 eingesetzt und der Dichtungsansatz 25 an seiner äußeren Mantelfläche 34 mit der inneren Mantelfläche des Hauptkörpers 6 an dessen proximalem Endbereich 16 verbunden. Hierzu wird bevorzugt eine lasergeschweißte Verbindung in der Kehle 36 gesetzt. Schließlich wird der Anschlusskörper 7 auf den Hauptkörper 6 aufgesetzt, wobei zum Herstellen der Klebeverbindung 38 Klebstoff am Ende des proximalen Endbereichs 16 aufgebracht wird. Der Anschlusskörper 7 wird mit dem Hauptkörper 6 erneut bevorzugt unter Ausbildung einer lasergeschweißten Verbindung verbunden. Hierzu wird beispielsweise an der Stoßstelle 44 eine umlaufende lasergeschweißte Naht gesetzt. Um das Arbeitskanalrohr 18 mit dem Anschlusskörper 7 zu verbinden, wird das chirurgische Instrument 2 erhitzt, so dass der eingebrachte Klebstoff aushärtet und die Klebeverbindung 38 bereitgestellt wird.

Alle genannten Merkmale, auch die den Zeichnungen allein zu entnehmenden sowie auch einzelne Merkmale, die in Kombination mit anderen Merkmalen offenbart sind, werden allein und in Kombination als offenbart angesehen. Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezuqszeichenliste

- 2: chirurgisches Instrument
- 4: Schaftrohr
- 6: Hauptkörper
- 7: Anschlusskörper
- 8: Behandlungsinstrument
- 10: Okular
- 12: Einführungsteil
- 14: Lichtleiteranschluss
- 16: proximaler Endbereich
- 18: Arbeitskanalrohr
- 20: erste O-Ringdichtung
- 22: Zwischenkörper
- 24: zweite O-Ringdichtung
- 25: Dichtungsansatz
- 26: Verbindungsabschnitt
- 28: Laserschweißlinie
- 30: Mantelabschnitt
- 32: Innenwandung
- 34: äußere Mantelfläche
- 36: Kehle
- 38: Klebeverbindung
- 40: Hohlraum
- 42: Dichtmittel
- 44: Stoßstelle
- 46: Zuführkanal
- 48: Arbeitskanal
- L: Längsrichtung
- R: Radialrichtung

## Patentansprüche

1. Chirurgisches Instrument (2) mit einem langgestreckten Schaftrohr (4), an welches sich ein Hauptkörper (6) anschließt, und mit einem Anschlusskörper (7), wobei der Anschlusskörper (7) mit einem proximalen Endbereich des Hauptkörpers (6) gekoppelt ist, und wobei in einem Innenraum des Schaftrohrs (4) zumindest ein Arbeitskanalrohr (18) angeordnet ist, welches sich in Längsrichtung (L) des Schaftrohrs (4) erstreckt, wobei ein proximaler Endbereich (16) des Arbeitskanalrohrs (18) innerhalb des Anschlusskörpers (7) aufgenommen und gegenüber diesem abgedichtet ist, **dadurch gekennzeichnet, dass** das Arbeitskanalrohr (18) mittels eines schaftförmigen Dichtungsansatzes (25) mit dem Hauptkörper (6) verbunden ist und der schaftförmige Dichtungsansatz (25) einen Mantelabschnitt (30) und einen Verbindungsabschnitt (26) umfasst, wobei der Verbindungsabschnitt (26) in einer radialen Richtung (R) eine größere Materialstärke aufweist als der Mantelabschnitt (30) und der Mantelabschnitt (30) mittels zumindest einer sich zumindest näherungsweise in Längsrichtung (L) erstreckenden linienförmigen Durchschweißverbindung mit dem Arbeitskanalrohr (18) verbunden ist.

2. Chirurgisches Instrument (2) nach Anspruch 1, **dadurch gekennzeichnet, dass** der proximale Endbereich (16) des Arbeitskanalrohrs (18) gegenüber dem Anschlusskörper (7) mittels einer Klebeverbindung (38) abgedichtet ist.

3. Chirurgisches Instrument (2) nach Anspruch 1 oder 2, **dadurch gekennzeichnet, dass** der Anschlusskörper (7) zumindest einen Zuführkanal (46) umfasst, der mit einem von dem Arbeitskanalrohr (18) umschlossenen Arbeitskanal (48) kommuniziert, insbesondere fluchtet, wobei der Endbereich (16) des Arbeitskanalrohrs (18) in dem Zuführkanal (46) des Anschlusskörpers (7) aufgenommen und mittels einer Klebeverbindung (38) zwischen einer Innenwandung des Zuführkanals (46) und einer Mantelfläche des Arbeitskanalrohrs (18) gegenüber dem Anschlusskörper (7) abgedichtet ist.

4. Chirurgisches Instrument (2) nach Anspruch 1, **dadurch gekennzeichnet, dass** der schaftförmige Dichtungsansatz (25) an seiner Innenwandung (32) mit einem proximalen Endbereich (16) des Arbeitskanalrohrs (18) und an seiner äußeren Mantelfläche (34) mit einem proximalen Endbereich des Hauptkörpers (6) verbunden ist.

5. Chirurgisches Instrument (2) nach einem der Ansprüche 1 bis 4, **dadurch gekennzeichnet, dass** die linienförmige Durchschweißverbindung eine lasergeschweißte Verbindung ist.

6. Chirurgisches Instrument (2) nach einem der Ansprüche 1 bis 5, **dadurch gekennzeichnet, dass** proximal des Dichtungsansatzes (25) zwischen Hauptkörper (6) und Anschlusskörper (7) ein an den Dichtungsansatz (25) angrenzender Hohlraum (40) im Anschlusskörper (7) vorhanden ist, wobei der proximale Endbereich (16) des Arbeitskanalrohrs (18) mit einer Klebeverbindung (38) mit dem Anschlusskörper (7) verbunden ist und der Hohlraum (40) mit einem Dichtmittel (42), insbesondere mit einem Dichtungsring, das zwischen einer äußeren Mantelfläche des Arbeitskanalrohrs (18) und dem Anschlusskörper (7) vorhanden ist, von der Klebeverbindung (38) getrennt ist, wobei die Klebeverbindung (38) durch einen unter Einwirkung von Wärme aushärtenden Klebstoff bereitgestellt wird.

7. Verfahren zum Herstellen eines chirurgischen Instruments (2) mit einem langgestreckten Schaftrohr (4), an welches sich ein Hauptkörper (6) anschließt, und mit einem Anschlusskörper (7), wobei der Anschlusskörper (7) mit einem proximalen Endbereich des Hauptkörpers (6) gekoppelt ist, und wobei in einem Innenraum des Schaftrohrs (4) zumindest ein Arbeitskanalrohr (18) angeordnet ist, welches sich in Längsrichtung (L) des Schaftrohrs (4) erstreckt, wobei ein proximaler Endbereich (16) des Arbeitskanalrohrs (18) innerhalb des Anschlusskörpers (7) aufgenommen und gegenüber diesem abgedichtet wird, **dadurch gekennzeichnet, dass** das Arbeitskanalrohr (18) mit einem schaftförmigen Dichtungsansatz (25) und der schaftförmigen Dichtungsansatz (25) mit dem Anschlusskörper (6) verbunden wird und der schaftförmige Dichtungsansatz (25) einen Mantelabschnitt (30) und einen Verbindungsabschnitt (26) umfasst, wobei der Verbindungsabschnitt (26) in einer radialen Richtung (R) eine größere Materialstärke aufweist als der Mantelabschnitt (30) und der Mantelabschnitt (30) mit zumindest einer sich zumindest näherungsweise in Längsrichtung (L) erstreckenden linienförmigen Durchschweißverbindung mit dem Arbeitskanalrohr (18) verbunden wird.

8. Verfahren nach Anspruch 7, **dadurch gekennzeichnet, dass** der proximale Endbereich (16) des Arbeitskanalrohrs (18) gegenüber dem Anschlusskörper (7) mit einer Klebeverbindung (38) abgedichtet wird.

9. Verfahren nach Anspruch 7 oder 8, **dadurch gekennzeichnet, dass** die linienförmige Durchschweißverbindung in einem Laserschweißverfahren hergestellt wird.

## Claims

1. A surgical instrument (2) with an elongated shaft tube (4), which is adjoined by a main body (6), and with a connection body (7), wherein the connection body (7) is coupled to a proximal end region of the main body (6) and wherein at least one working channel tube (18) is arranged in an interior of the shaft tube (4), which working channel tube (18) extends in a longitudinal direction (L) of the shaft tube (4), wherein a proximal end region (16) of the working channel tube (18) is accommodated inside the connection body (7) and is sealed with respect thereto, **characterized in that** the working channel tube (18) is connected to the main body (6) by means of a shaft-shaped seal shoulder (25) and the shaft-shaped seal shoulder (25) comprises a jacket section (30) and a connecting section (26), wherein the connecting section (26) has a greater material thickness in a radial direction (R) than the jacket section (30), and the jacket section (30) is connected to the working channel tube (18) by means of at least one linear through-weld connection that extends at least approximately in the longitudinal direction (L).

2. The surgical instrument (2) according to claim 1, **characterized in that** the proximal end region (16) of the working channel tube (18) is sealed with respect to the connection body (7) by means of an adhesive connection (38).

3. The surgical instrument (2) according to claim 1 or 2, **characterized in that** the connection body (7) comprises at least one feed channel (46) that communicates, in particular aligns, with a working channel (48) that is surrounded by the working channel tube (18), wherein the end region (16) of the working channel tube (18) is accommodated in the feed channel (46) of the connection body (7) and is sealed with respect to the connection body (7) by means of an adhesive connection (38) between an inner wall of the feed channel (46) and a jacket surface of the working channel tube (18).

4. The surgical instrument (2) according to claim 1, **characterized in that** the shaft-shaped seal shoulder (25) is connected on its inner wall (32) to a proximal end region (16) of the working channel tube (18) and on its outer jacket surface (34) to a proximal end region of the main body (6).

5. The surgical instrument (2) according to one of claims 1 to 4, **characterized in that** the linear through-weld connection is a laser-welded connection.

6. The surgical instrument (2) according to one of claims 1 to 5, **characterized in that** a hollow space (40) adjacent to the seal shoulder (25) is present in the connection body (7) proximal to the seal shoulder (25) between the main body (6) and the connection body (7), wherein the proximal end region (16) of the working channel tube (18) is connected to the connection body (7) with an adhesive connection (38) and the hollow space (40) is separated from the adhesive connection (38) by a sealing means (42), in particular a sealing ring that is present between an outer jacket surface of the working channel tube (18) and the connection body (7), wherein the adhesive connection (38) is provided by an adhesive that is hardened under the influence of heat.

7. A method for producing a surgical instrument (2) with an elongated shaft tube (4), which is adjoined by a main body (6), and with a connection body (7), wherein the connection body (7) is coupled to a proximal end region of the main body (6) and wherein at least one working channel tube (18) is arranged in an interior of the shaft tube (4), which working channel tube (18) extends in a longitudinal direction (L) of the shaft tube (4), wherein a proximal end region (16) of the working channel tube (18) is accommodated inside the connection body (7) and is sealed with respect thereto, **characterized in that** the working channel tube (18) is connected to a shaft-shaped seal shoulder (25) and the shaft-shaped seal shoulder (25) is connected to the connection body (6) and the shaft-shaped seal shoulder (25) comprises a jacket section (30) and a connecting section (26), wherein the connecting section (26) has a greater material thickness in a radial direction (R) than the jacket section (30), and the jacket section (30) is connected to the working channel tube (18) with at least one linear through-weld connection that extends at least approximately in the longitudinal direction (L).

8. The method according to claim 7, **characterized in that** the proximal end region (16) of the working channel tube (18) is sealed with respect to the connection body (7) with an adhesive connection (38).

9. The method according to claim 7 or 8, **characterized in that** the linear through-weld connection is produced with a laser welding method.

## Revendications

1. Instrument chirurgical (2) comprenant un tube allongé (4) formant tige, auquel un corps principal (6) est connecté, et un corps de liaison (7), le corps de liaison (7) étant relié à une partie d'extrémité proximale du corps principal (6), et au moins une conduite de travail (18) étant agencée dans un espace intérieur du tube (4) formant tige, laquelle conduite (18) s'étend dans la direction longitudinale (L) du tube (4) formant tige, une partie d'extrémité proximale (16) de la conduite de travail (18) étant reçue à l'intérieur du corps de liaison (7) et étant rendue étanche par rapport à celui-ci, **caractérisé en ce que** la conduite de travail (18) est reliée au corps principal (6) au moyen d'un embout d'étanchéité (25) en forme de tige, et l'embout d'étanchéité (25) en forme de tige comprend une portion d'enveloppe (30) et une portion de raccordement (26), la portion de raccordement (26) présentant une épaisseur de matériau dans une direction radiale (R) plus importante que la portion d'enveloppe (30), et la portion d'enveloppe (30) étant reliée à la conduite de travail (18) au moyen d'au moins une liaison soudée linéaire qui s'étend au moins approximativement dans la direction longitudinale (L).

2. Instrument chirurgical (2) selon la revendication 1, **caractérisé en ce que** la partie d'extrémité proximale (16) de la conduite de travail (18) est rendue étanche par rapport au corps de liaison (7) au moyen d'une liaison adhésive (38).

3. Instrument chirurgical (2) selon la revendication 1 ou la revendication 2, **caractérisé en ce que** le corps de liaison (7) comprend au moins un conduit d'alimentation (46) qui communique, en particulier qui est aligné, avec un conduit de travail (48) entouré par la conduite de travail (18), la partie d'extrémité (16) de la conduite de travail (18) étant reçue dans le conduit d'alimentation (46) du corps de liaison (7) et est rendue étanche par rapport au corps de liaison (7) au moyen d'une liaison adhésive (38) entre une paroi intérieure du conduit d'alimentation (46) et une surface d'enveloppe de la conduite de travail (18).

4. Instrument chirurgical (2) selon la revendication 1, **caractérisé en ce que** l'embout d'étanchéité (25) en forme de tige est relié sur sa paroi intérieure (32) à une partie d'extrémité proximale (16) de la conduite de travail (18) et sur sa surface circonférentielle extérieure (34) à une partie d'extrémité proximale du corps principal (6).

5. Instrument chirurgical (2) selon l'une quelconque des revendications 1 à 4, **caractérisé en ce que** la liaison soudée linéaire est une jonction soudée au laser.

6. Instrument chirurgical (2) selon l'une des revendications 1 à 5, **caractérisé en ce que** dans le corps de liaison (7), à proximité de l'embout d'étanchéité (25), est prévu un espace creux (40) se raccordant à l'embout d'étanchéité (25) entre le corps principal (6) et le corps de liaison (7), la partie d'extrémité proximale (16) de la conduite de travail (18) étant reliée au corps de liaison (7) par une liaison adhésive (38), et l'espace creux (40) est séparé de la jonction adhésive (38) par un moyen d'étanchéité (42), en particulier une bague d'étanchéité, qui est présente entre une surface circonférentielle extérieure de la conduite de travail (18) et le corps de liaison (7), la liaison adhésive (38) étant assurée par un adhésif qui durcit sous l'action de la chaleur.

7. Un procédé de fabrication d'un instrument chirurgical (2) comprenant un tube allongé (4) formant tige, auquel un corps principal (6) est connecté, et un corps de liaison (7), le corps de liaison (7) étant relié à une partie d'extrémité proximale du corps principal (6), et au moins un conduite de travail (18) étant agencée dans un espace intérieur du tube (4) formant tige, laquelle conduite (18) s'étend dans la direction longitudinale (L) du tube (4) formant tige, une partie d'extrémité proximale (16) de la conduite de travail (18) étant reçue à l'intérieur du corps de liaison (7) et étant rendue étanche par rapport à celui-ci, caractérisé en ce en ce que la conduite de travail (18) est reliée au corps de raccordement (6) au moyen un embout d'étanchéité (25) en forme de tige et l'embout d'étanchéité (25) en forme de tige est relié au corps de raccordement (6), et l'embout d'étanchéité (25) en forme de tige comprend une portion d'enveloppe (30) et une portion de raccordement (26), la section de raccordement (26) présentant une épaisseur de matériau dans une direction radiale (R) plus importante que la portion d'enveloppe (30) et la portion d'enveloppe (30) étant reliée à la conduite de travail (18) au moyen d'au moins une liaison soudée linéaire qui s'étend au moins approximativement dans la direction longitudinale (L).

8. Le procédé selon la revendication 7, **caractérisé en ce que** la partie d'extrémité proximale (16) de la conduite de travail (18) est rendue étanche par rapport au corps de liaison (7) au moyen d'une liaison adhésive (38).

9. Le procédé selon la revendication 7 ou la revendication 8, **caractérisé en ce que** la liaison soudée linéaire est produite au moyen d'un procédé de soudage au laser.
